(19) Europäisches Patentamt / European Patent Office / Office européen des brevets

(11) **EP 3 730 936 B1**

(12) **EUROPEAN PATENT SPECIFICATION**

(45) Date of publication and mention
of the grant of the patent:
**11.10.2023 Bulletin 2023/41**

(21) Application number: **18889992.6**

(22) Date of filing: **17.12.2018**

(51) International Patent Classification (IPC):
**G01N 33/06** (2006.01)    **A23L 33/00** (2016.01)

(52) Cooperative Patent Classification (CPC):
**A23L 33/40; G01N 33/06**

(86) International application number:
**PCT/CN2018/121493**

(87) International publication number:
**WO 2019/120166 (27.06.2019 Gazette 2019/26)**

(54) **METHOD FOR EVALUATING SIMILARITY OF HUMAN MILK SUBSTITUTE FAT**

VERFAHREN ZUR BEWERTUNG DER ÄHNLICHKEIT VON MENSCHLICHEM MILCHERSATZFETT

PROCÉDÉ D'ÉVALUATION DE SIMILARITÉ DE MATIÈRE GRASSE DE SUBSTITUT DE LAIT HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2017 CN 201711392850**

(43) Date of publication of application:
**28.10.2020 Bulletin 2020/44**

(73) Proprietor: **Jiangnan University**
**Wuxi, Jiangsu 214122 (CN)**

(72) Inventors:
• **WANG, Xingguo**
  **Wuxi, Jiangsu 214122 (CN)**
• **WEI, Wei**
  **Wuxi, Jiangsu 214122 (CN)**
• **JIN, Qingzhe**
  **Wuxi, Jiangsu 214122 (CN)**

(74) Representative: **karo IP**
**karo IP Patentanwälte**
**Kahlhöfer Rößler Kreuels PartG mbB**
**Platz der Ideen 2**
**40476 Düsseldorf (DE)**

(56) References cited:
EP-A2- 0 209 327      CN-A- 104 855 542
CN-A- 105 028 659      CN-A- 106 814 171
CN-A- 108 132 338

• ZOU XIAOQIANG ET AL: "Preparation and Characterization of Human Milk Fat Substitutes Based on Triacylglycerol Profiles", JOURNAL OF THE AMERICAN OIL CHEMISTS' SOCIETY (JAOCS), SPRINGER, DE, vol. 93, no. 6, 3 May 2016 (2016-05-03), pages 781-792, XP035947600, ISSN: 0003-021X, DOI: 10.1007/S11746-016-2816-7 [retrieved on 2016-05-03]
• ZOU XIAOQIANG: "Model for Human Milk Fat Substitute Evaluation Based on Triacylglycerol Composition Profile", JOURNAL OF AGRICULTURE AND FOOD CHEMISTRY, vol. 61, no. 1, 10 December 2012 (2012-12-10), pages 167-175, XP055622068,
• WANG YONGHUA: "Establishment of an Evaluation Model for Human Milk Fat Substitutes", JOURNAL OF AGRICULTURE AND FOOD CHEMISTRY, vol. 58, no. 1, 31 December 2010 (2010-12-31), pages 642-649, XP055622071,

EP 3 730 936 B1

**EP 3 730 936 B1**

**Description**

BACKGROUND OF THE INVENTION

1. TECHNICAL FIELD

This invention generally relates to infant formula food products field, and more specifically to a method for determining the similarity of the fat of infant formula food products to human milk fat.

2. BACKGROUND ART

Human milk is commonly considered as the best food for infants because it contains numerous functional constituents for infants' growth and development. Throughout the development of infant formula, it is a process history for mimicking human milk, especially with respect to fat. The fat used in infant formula food belongs to human milk fat substitutes. It was initially produced according to the ratio of fat in human milk, then the essential fatty acids and functional long-chain polyunsaturated fatty acids such as docosahexaenoic acid (DHA), arachidonic acid (AA), were added to infant formulas based on the fatty acid composition and distribution of human milk fat. The ultimate aim of human milk fat substitutes is to mimic the chemical structure including fatty acids, triacylglycerols and complicated lipids composition, and physical properties including milk fat globule and sensory characteristics of human milk fat as close as possible.

So far there is no method for evaluating human milk fat substitute in which the lipid composition of human milk fat is thoroughly considered. For example, no method is available presently for the nutrients that are important for the intellectual development of infants but with very low content in human milk fat. Therefore, we need an objective, scientific and comprehensive evaluation method for quantifying the similarity between human milk fat substitute and human milk fat. Meanwhile, it is necessary to establish a comprehensive evaluation model to consider all aspects of the lipid composition of human milk fat and the whole similarity of human milk fat substitute.

ZOU XIAOQIANG ET AL: "Preparation and Characterization of Human Milk Fat Substitutes Based on Triacylglycerol Profiles", JOURNAL OF THE AMERICAN OIL CHEMISTS' SOCIETY (JAOCS), SPRINGER, DE, vol. 93, no. 6, 3 May 2016 (2016-05-03), pages 781-792, disclose a similarity evaluation between human milk fat and human milk fat substitutes.

ZOU XIAOOIANG ET AL: "Model for Human Milk Fat Substitute Evaluation Based on Triacylglycerol Composition Profile", JOURNAL OF AGRICULTURE AND FOOD CHEMISTRY, vol. 61, no. 1, 10 December 2012 (2012-12-10), pages 167-175, disclose a similarity evaluation between human milk fat and human milk fat substitutes.

SUMMARY OF THE INVENTION

The present invention provides an objective, scientific and comprehensive method for similarity evaluation of dietary fat, and briefly introduces some better embodiments. Perhaps it should be noted that some simplifications or omissions may be made in the present context to make it clear, but such simplifications or omissions may not be used to limit the scope of the present invention.

In view of the problems in the above and/or existing evaluation models for dietary fat, the present invention is proposed.

The purpose of the present invention is to fill in the gaps in the prior art and to provide an evaluation method that takes into account all aspects of lipid compositions comprehensively.

The present invention provides improved procedures for evaluating the similarity of dietary fat to human milk fat, wherein the composition of major fatty acids, trace fatty acids, major sn-2 fatty acids, trace sn-2 fatty acids, major triacylglycerols and trace triacylglycerols is analysed, and wherein the major fatty acids are the fatty acid that represents more than 1% of the total fatty acids; major sn-2 fatty acids are the fatty acids that represent more than 1% of the total sn-2 fatty acids; major triacylglycerols are the triacylglycerols that represent more than 1% of the total triacylglycerols; trace fatty acids are defined as the fatty acids with less than 1% of the total fatty acids, except for special fatty acids; trace sn-2 fatty acids are defined as the fatty acids with less than 1% of the total sn-2 fatty acids, except for special fatty acids; trace triacylglycerols are defined as the triacylglycerols with less than 1% of the triacylglycerols; special fatty acids are fatty acids that are different from conventional fatty acids due to their special structure of functional group contain odd-chain fatty acids, branched-chain fatty acids, cyclic fatty acids, alkenyl acid.

The actual values of a certain index in dietary fat are compared with the range of standard values of the corresponding index in human milk fat to calculate the drift rate of each index of dietary fat by the use of the equation:

$$C = \frac{|b - a|}{a}$$

in which, a represents the standard value of an index, b represents the actual value of the index. If b is more than amax, a is equivalent to amax; and if b is less than amin, a is equivalent to amin. C, represents the extent of the determined value of a certain index deviating from the theoretical value range, i.e. shift rate; When b is between amin and amax, C is defined as 0, namely, shift rate is equivalent to 0.

a similarity evaluation model is established using the equation:

$$G = \sum_{i=1}^{n} \left[ (1 - \frac{|b-a|}{a}) \times 100 \right] \times \frac{d_i}{\sum_{i=1}^{n} d_i}$$

where $d_i$ represents the appointed weight for i$^{th}$ index; $d_i \Big/ \sum_{i=1}^{n} d_i$ represents the normalized weight for the i$^{th}$ index after normalization; G represents the similarity coefficient of a certain index of dietary fat; the higher G value, the higher the similarity; and the maximum of G value is 100; conversely, the smaller G value, the lower similarity.

What is claimed is that it comprises conducting a hierarchical evaluation of multi-grade indexes of dietary fat to obtain the similarity through the model as follow:

$$G_{III} = \sum_{i=1}^{n} \left[ (1 - \frac{|b-a|}{a}) \times 100 \right] \times \frac{d_i}{\sum_{i=1}^{n} d_i}$$

$$G_{II} = \sum_{i=1}^{n} e_i G_{III}$$

$$G_I = \sum_{i=1}^{n} f_i G_{II}$$

in which, $G_{III}$, represents the similarity coefficient of each grade III index of dietary fat to human milk fat; $G_{II}$ represents the similarity coefficient of each grade II index of dietary fat to human milk fat; $e_i$ represents the appointed weight for the i$^{th}$ grade III index, $0 \le e_i \le 1$; $G_I$, represents the similarity coefficient of all grade II index of dietary fat to human milk fat; $f_i$ represents the appointed weight for the i$^{th}$ grade II index, $0 \le f_i \le 1$.

The standard value of a certain index, a, is expressed as mean $\pm$ n times of standard deviation ($a = \bar{a} + n\sigma$). Preferably, a is the 95% confidence interval, n =1.96.

With respect to the application of the method of this patent disclosure to dietary fat, it should be specifically noted that the grade I index is the lipid composition of human milk fat.

The grade II index contains fatty acids, *sn*-2 fatty acids, triacylglycerols and complicated lipids.

The fatty acids in grade III index contain major fatty acids, trace fatty acids and special fatty acids; The *sn*-2 fatty acids in grade III index comprises major *sn*-2 fatty acids, trace *sn*-2 fatty acids and special *sn*-2 fatty acids.

The triacylglycerols in grade III index contain major triacylglycerols and trace triacylglycerols.

The complicated lipids in grade III index contain phospholipids, sterols, and glycolipids.

The major fatty acids are the fatty acid that represents more than 1% of the total fatty acids; major *sn*-2 fatty acids are the fatty acids that represent more than 1% of the total *sn*-2 fatty acids; major triacylglycerols are the triacylglycerols that represent more than 1% of the total triacylglycerols; trace fatty acids are defined as the fatty acids with less than 1% of the total fatty acids, except for special fatty acids; trace *sn*-2 fatty acids are defined as the fatty acids with less than 1% of the total *sn*-2 fatty acids, except for special fatty acids; trace triacylglycerols are defined as the triacylglycerols with less than 1% of the triacylglycerols; special fatty acids are fatty acids that are different from conventional fatty acids due to their special structure of functional group contain odd-chain fatty acids, branched-chain fatty acids, cyclic fatty acids, alkenyl acid.

BRIEF DESCRIPTION OF THE DRAWINGS

In order to illustrate the of this invention, the preparation of the appended drawings used in the below embodiments is described. Obviously, those skilled in the art will appreciate that other drawings may be obtained according to these without creative labour.

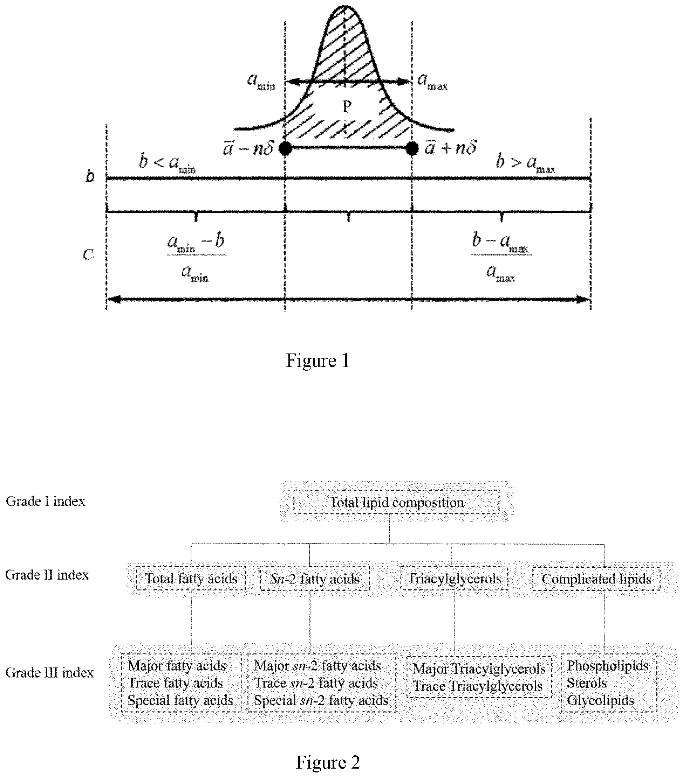

FIG.1 depicts the content distribution of a certain index in human milk fat. When the sample size is adequately large, the content appears in the normal distribution.
FIG.2 shows the schematic of the hierarchical evaluation system for the similarity of multistage indexes between dietary fat and human milk fat.

DESCRIPTION OF PREFERRED EMBODIMENTS

The following examples serve to illustrate the application of this invention in combination with the appended drawings of the specification. It is obvious that the preferred embodiments of this invention are illustrated, but should not be limited by the following examples.

Stated in more detailed process terms, some of the more preferred embodiments of the invention may be illustrated, but should not be limited by the following additional exemplary versions of the herein disclosed processes. The process of this invention can also be implemented in other ways different from those described herein.

"An embodiment" or "an example" herein means that a particular feature, structure or property may be contained in at least one implementation of the present invention. "In one embodiment" appearing in different places in this specification does not mean the same embodiment, nor an embodiment that is exclusive or selective from other embodiments.

The following examples serve to illustrate the process of the invention to evaluate the similarity of dietary fat to human milk fat. The data of the lipid composition of 309 human milk samples in Wuxi, China, with the 95% confidence interval, is used as the evaluation basis. In the calculation of 95% confidence interval of human milk fat index, some indexes fail to show the normal distribution and some have negative boundary value. However, all the index data on human milk fat, biological samples with a large enough sample size, should follow the normal distribution. Considering the continuity of similarity evaluation, the calculation method of confidence interval under normal distribution is still selected in the examples, and the boundary value of the confidence interval is set to 0 when it is negative.

Based on the existing analysis methods, similarity evaluation is conducted mainly based on the composition of major fatty acids, trace fatty acids, major *sn*-2 fatty acids, trace *sn*-2 fatty acids, major triacylglycerols and trace triacylglycerols in below examples.

The contents of fatty acids, *sn*-2 fatty acids and triacylglycerols of human milk fat (95% confidence interval) are shown in Table 1~3.

Table 1. The content of major and trace fatty acids of human milk fat (95% confidence interval)

| Major fatty acids | Range | Trace fatty acids | Range | Trace fatty acids | Range |
|---|---|---|---|---|---|
| 10:0 | 0.06~2.21 | 6:0 | 0~0.10 | 18:3(n-6) | 0~0.23 |
| 12:0 | 1.19~8.51 | 8:0 | 0.03~0.33 | 20:2(n-6) | 0.12~1.63 |
| 14:0 | 2.20~7.93 | 20:0 | 0.05~0.40 | 20:3(n-6) | 0.22~0.97 |
| 16:0 | 15.07~22.62 | 22:0 | 0~0.27 | 20:4(n-6) | 0.37~1.31 |
| 16:1(n-7) | 0.98~2.39 | 24:0 | 0~0.21 | 20:5(n-3) | 0~0.30 |
| 18:0 | 3.67~8.32 | 14:1(n-5) | 0.02~0.34 | 22:2(n-6) | 0~0.46 |
| 18:1(n-9) | 26.04~37.27 | 20:1(n-9) | 0.22~1.15 | 22:4(n-6) | 0~0.50 |
| 18:2(n-6) | 14.19~25.52 | 22:1(n-9) | 0~0.40 | 22:5(n-3) | 0~0.66 |
| 18:3(n-3) | 0.68~1.73 | 24:1(n-9) | 0~0.34 | 22:5(n-6) | 0.02~0.4 |
| | | | | 22:6(n-3) | 0.22~0.95 |

Table 2. The content of major and trace *sn*-2 fatty acids of human milk fat (95% confidence interval)

| Major *sn*-2 fatty acids | Range | Trace *sn*-2 fatty acids | Range | Trace *sn*-2 fatty acids | Range |
|---|---|---|---|---|---|
| 12:0 | 0.65~10.24 | 6:0 | 0~0.09 | 18:3(n-3) | 0.26~1.23 |
| 14:0 | 4.46~15.52 | 8:0 | 0~0.38 | 18:3(n-6) | 0~0.14 |
| 16:0 | 43.31~58.37 | 10:0 | 0.10~1.36 | 20:2(n-6) | 0.13~0.74 |
| 16:1(n-7) | 1.37~2.74 | 20:0 | 0.12~0.31 | 20:3(n-6) | 0.12~0.66 |
| 18:0 | 1.21~2.56 | 22:0 | 0~0.28 | 20:4(n-6) | 0.43~1.22 |
| 18:1(n-9) | 6.98~17.23 | 24:0 | 0~0.15 | 20:5(n-3) | 0~0.23 |
| 18:2(n-6) | 3.31~13.42 | 14:1(n-5) | 0.02~0.27 | 22:2(n-6) | 0.02~0.59 |
| | | 20:1(n-9) | 0.24~0.70 | 22:4(n-6) | 0~0.60 |
| | | 22:1(n-9) | 0~0.26 | 22:5(n-3) | 0.02~0.70 |
| | | 24:1(n-9) | 0~0.60 | 22:5(n-6) | 0.04~0.51 |
| | | | | C22:6(n-3) | 0.59~1.47 |

Table 3. The content of major and trace triacylglycerol of human milk fat (95% confidence interval)

| Major triacylglycerols | Range | Major triacylglycerols | Range | Trace triacylglycerols | Range |
|---|---|---|---|---|---|
| LaLaO | 0~2.21 | POLa | 4.35~10.78 | CaLaLa | 0~0.92 |
| CaPL | 0~4.88 | POL | 17.85 ~ 33.02 | BuOP | 0~0.51 |
| LLL | 0~3.49 | PPL | 1.57~7.25 | MLaCa | 0~0.47 |
| LaOL | 0.30 ~ 7.20 | MPO | 0.36~3.17 | CaLaL | 0~0.36 |
| CaPO | 0~8.52 | OOO | 0.52~3.73 | CaLaO | 0~1.60 |
| OLL | 2.90 ~ 10.35 | POO | 6.81~24.86 | LaLaM | 0~1.15 |
| PLL | 0~11.39 | PPO | 1.56~8.07 | SOO | 0~1.45 |
| MOL | 0~13.58 | POS | 0~2.83 | PPS | 0.04 ~ 1.16 |
| LaOO | 0.21 ~ 3.42 | | | | |

## EXAMPLE 1

The similarity coefficients of 4 human milk samples, referred to as HM1, HM2, HM3 and HM4, are evaluated with the use of the above evaluation model. The results of fatty acid, *sn*-2 fatty acid and triacylglycerol composition are shown in Table 4~6.

Table 4. Fatty acids composition of human milk samples (n=4)

| Fatty acids | HM1 | HM2 | HM3 | HM4 |
|---|---|---|---|---|
| 6:0 | 0.01 | 0.03 | 0.04 | 0.04 |
| 8:0 | 0.17 | 0.14 | 0.14 | 0.13 |
| 10:0 | 1.15 | 1.39 | 1.39 | 0.76 |
| 12:0 | 3.85 | 5.44 | 5.67 | 2.34 |
| 14:0 | 2.85 | 4.58 | 4.79 | 2.48 |

(continued)

| Fatty acids | HM1 | HM2 | HM3 | HM4 |
|---|---|---|---|---|
| 14:1(n-5) | 0.02 | 0.03 | 0.03 | 0.05 |
| 16:0 | 17.04 | 19.52 | 19.59 | 22.28 |
| 16:1(n-7) | 1.64 | 1.71 | 1.72 | 2.32 |
| 18:0 | 4.93 | 5.53 | 5.60 | 6.85 |
| 18:1(n-9) | 30.26 | 31.47 | 31.04 | 35.60 |
| 18:2(n-6) | 30.12 | 21.81 | 21.72 | 18.40 |
| 18:3(n-6) | 0.18 | 0.20 | 0.20 | 0.26 |
| 18:3(n-3) | 0.97 | 1.68 | 1.68 | 1.15 |
| 20:0 | 0.15 | 0.18 | 0.17 | 0.15 |
| 20:1(n-9) | 0.39 | 0.47 | 0.48 | 0.60 |
| 20:2(n-6) | 0.56 | 0.51 | 0.51 | 0.40 |
| 20:3(n-6) | 0.59 | 0.47 | 0.48 | 0.39 |
| 20:4(n-6) | 0.86 | 0.64 | 0.65 | 0.61 |
| 20:3(n-3) | 0.07 | 0.08 | 0.08 | 0.07 |
| 22:0 | 0.08 | 0.06 | 0.06 | 0.03 |
| 22:1(n-9) | 0.12 | 0.18 | 0.19 | 0.26 |
| 20:5(n-3) | 0.09 | 0.04 | 0.04 | 0.15 |
| 22:2(n-6) | 0.07 | 0.07 | 0.07 | 0.03 |
| 22:4(n-6) | 0.27 | 0.20 | 0.20 | 0.15 |
| 22:5(n-6) | 0.17 | 0.10 | 0.10 | 0.14 |
| 24:1(n-9) | 0.03 | 0.05 | 0.05 | 0.02 |
| 22:5(n-3) | 0.22 | 0.12 | 0.12 | 0.22 |
| 22:6(n-3) | 0.58 | 0.34 | 0.34 | 0.81 |

Table 5. *Sn*-2 fatty acids composition of human milk samples (n=4)

| Fatty acids | HMF1 | HMF2 | HMF3 | HMF4 |
|---|---|---|---|---|
| 10:0 | 0.85 | 0.87 | 0.43 | 0.55 |
| 12:0 | 7.14 | 7.29 | 3.16 | 6.60 |
| 14:0 | 8.93 | 9.04 | 4.92 | 10.70 |
| 14:1(n-5) | 0.03 | 0.02 | 0.02 | 0.01 |
| 16:0 | 47.31 | 46.18 | 53.33 | 46.79 |
| 16:1(n-7) | 2.28 | 2.27 | 2.73 | 2.31 |
| 18:0 | 1.72 | 1.71 | 2.34 | 2.64 |
| 18:1(n-9) | 11.80 | 12.04 | 13.81 | 9.58 |
| 18:2(n-6) | 13.69 | 14.14 | 12.10 | 13.58 |
| 18:3(n-6) | 0.12 | 0.13 | 0.17 | 0.06 |
| 18:3(n-3) | 1.02 | 1.16 | 0.96 | 1.19 |

(continued)

| Fatty acids | HMF1 | HMF2 | HMF3 | HMF4 |
|---|---|---|---|---|
| 20:0 | 0.19 | 0.19 | 0.14 | 0.12 |
| 20:1(n-9) | 0.29 | 0.29 | 0.28 | 0.25 |
| 20:2(n-6) | 0.30 | 0.32 | 0.20 | 0.15 |
| 20:3(n-6) | 0.24 | 0.24 | 0.16 | 0.12 |
| 20:4(n-6) | 0.55 | 0.56 | 0.43 | 0.48 |
| 22:0 | 0.06 | 0.06 | 0.03 | 0.04 |
| 22:1(n-9) | 0.14 | 0.14 | 0.05 | 0.06 |
| 20:5(n-3) | 0.01 | 0.01 | 0.06 | 0.03 |
| 22:2(n-6) | 0.15 | 0.16 | 0.06 | 0.06 |
| 22:4(n-6) | 0.36 | 0.38 | 0.28 | 0.22 |
| 22:5(n-6) | 0.19 | 0.20 | 0.24 | 0.12 |
| 22:5(n-3) | 0.26 | 0.28 | 0.45 | 0.34 |
| 22:6(n-3) | 0.57 | 0.61 | 1.29 | 0.99 |

Table 6. Triacylglycerols composition of human milk samples (n=4)

| Triacylglycerols | HM1 | HM2 | HM3 | HM4 |
|---|---|---|---|---|
| CaLaLa | 0.2 | 0.26 | 0.17 | 0.07 |
| BuPO | 0.31 | 0.11 | 0.07 | 0.36 |
| CaLaM | 0.22 | 0.18 | 0.15 | 0.23 |
| CaLaL | 0.12 | 0.14 | 0.11 | 0.08 |
| LaCaO | 1.28 | 0.58 | 0.58 | 0.42 |
| LaLaM | 0.45 | 0.99 | 0.57 | 0.34 |
| LaLaO | 1.82 | 0.26 | 0.9 | 0.59 |
| CaPL | 5.03 | 2.47 | 1.54 | 2.02 |
| LLL | 1.96 | 1.06 | 1.94 | 1.57 |
| LaOL | 5.45 | 2.18 | 4.59 | 2.42 |
| CaPO | 4.19 | 4.63 | 4.62 | 3.07 |
| OLL | 6.48 | 8.49 | 8.57 | 6.34 |
| PLL | 3.75 | 2.03 | 5.46 | 7.43 |
| MOL | 5.42 | 7.93 | 5.06 | 3.9 |
| LaOO | 1.63 | 2.19 | 1.36 | 0.85 |
| LaPO | 8.2 | 6.67 | 6.46 | 7.33 |
| OPL | 23.27 | 29.36 | 24.5 | 25.63 |
| PPL | 3.25 | 4.62 | 2.09 | 3.35 |
| MPO | 1.43 | 1.77 | 1.55 | 1.93 |
| OOO | 1.46 | 1.85 | 2.46 | 1.94 |
| OPO | 16.85 | 15.92 | 18.46 | 20.8 |

(continued)

| Triacylglycerols | HM1 | HM2 | HM3 | HM4 |
|---|---|---|---|---|
| PPO | 4.13 | 3.57 | 4.24 | 3.43 |
| SOO | 0.14 | 0.23 | 0.28 | 0.33 |
| POS | 1.46 | 1.26 | 1.3 | 2.14 |
| PPS | 0.86 | 0.56 | 0.78 | 0.97 |

The similarity coefficients of 4 human milk samples evaluated with the use of the above evaluation model are shown in Table 7.

Table 7. Similarity coefficients of human milk samples (n=4)

| | $G_{III}$ | | | | | | $G_{II}$ | | | $G_I$ |
|---|---|---|---|---|---|---|---|---|---|---|
| | $G_{MAFA}$ | $G_{MIFA}$ | $G_{sn\text{-}2MAFA}$ | $G_{sn\text{-}2MIFA}$ | $G_{MATAG}$ | $G_{MITAG}$ | $G_{FA}$ | $G_{sn\text{-}2FA}$ | $G_{TAG}$ | $G$ |
| HM1 | 98.87 | 100.00 | 99.71 | 99.84 | 99.82 | 100.00 | 99.43 | 99.78 | 99.91 | 99.71 |
| HM2 | 100.00 | 100.00 | 99.23 | 100.00 | 100.00 | 100.00 | 100.00 | 99.62 | 100.00 | 99.87 |
| HM3 | 100.00 | 100.00 | 100.00 | 98.84 | 100.00 | 100.00 | 100.00 | 99.42 | 100.00 | 99.81 |
| HM4 | 100.00 | 99.36 | 99.39 | 97.62 | 100.00 | 100.00 | 99.68 | 98.50 | 100.00 | 99.39 |

The results indicate that the similarity coefficients of 4 human milk samples in each grade indexes are very high, which reflect the accuracy of evaluation model of this patent to some extent.

EXAMPLE 2

The similarity coefficients of 2 kinds of human milk fat substrates that have been used in infant formulas as reported, referred to as IF-A (19% of total palmitic acid located at *sn-2* position) and IF-B (44.5% of total palmitic acid located at *sn-2* position) respectively, are evaluated with the use of the above evaluation model. Clinical trials have shown that IF-B could reduce the loss of total fatty acids and palmitic acid in the faeces of infants compared with IF-A.
The results of total fatty acids and *sn-2* fatty acids composition of 2 kinds of human milk fat substrates are given in Table 8~9 below.

Table 8. Fatty acids composition of 2 kinds of human milk fat substrates

| Fatty acids | IF-A | IF-B |
|---|---|---|
| 4:0 | 0.16 | 0.05 |
| 6:0 | 0.24 | 0.1 |
| 8:0 | 0.75 | 0.56 |
| 10:0 | 1.14 | 0.74 |
| 12:0 | 5.57 | 7.74 |
| 13:0 | 0.02 | 0.01 |
| 14:0 | 5.11 | 4.39 |
| 14:1(n-5) | 0.27 | 0.09 |
| 15:0 | 0.35 | 0.16 |
| 15:1 | 0.09 | 0.04 |
| 16:0 | 23.86 | 23.87 |
| 16:1(n-9) | 0.13 | 0.11 |
| 16:1(n-7) | 0.78 | 0.45 |

(continued)

| Fatty acids | IF-A | IF-B |
|---|---|---|
| 17:0 | 0.3 | 0.17 |
| 17:1 | 0.14 | 0.07 |
| 18:0 | 6.72 | 4.55 |
| 18:1(n-9) | 38.09 | 40.4 |
| 18:2(n-6) | 13.55 | 13.88 |
| 20:0 | 0.38 | 0.26 |
| 18:3(n-3) | 1.07 | 1.2 |
| 20:1(n-9) | 0.45 | 0.45 |
| 20:2(n-6) | 0.06 | 0.06 |
| 20:3(n-6) | 0.04 | 0.03 |
| 20:4(n-6) | 0.2 | 0.19 |
| 22:0 | 0.25 | 0.15 |
| 24:0 | 0.11 | 0.08 |
| 22:4(n-6) | 0.02 | 0.02 |
| 22:5(n-6) | 0.03 | 0.03 |
| 22:5(n-3) | 0.04 | 0.03 |
| 22:6(n-3) | 0.11 | 0.12 |

Table 9. $Sn$-2 fatty acids composition of 2 kinds of human milk fat substrates

| Fatty acids | IF-A | IF-B |
|---|---|---|
| 12:0 | 8.36 | 10.87 |
| 14:0 | 6.39 | 5.65 |
| 16:0 | 13.59 | 31.88 |
| 18:0 | 2.06 | 2.02 |
| 18:1(n-9) | 52.08 | 32.64 |
| 18:2(n-6) | 16.15 | 15.35 |

The similarity coefficients of 2 kinds of human milk fat substrates evaluated with the use of the above evaluation model are given in Table 10 below.

Table 10. Similarity coefficients of 2 kinds of human milk fat substrates

| | $G_{III}$ | | | $G_{II}$ | | $G_I$ |
|---|---|---|---|---|---|---|
| | $G_{MAFA}$ | $G_{MIFA}$ | $G_{sn-2MAFA}$ | $G_{FA}$ | $G_{sn-2FA}$ | $G$ |
| IF-A | 96.38 | 73.95 | 44.10 | 85.16 | 44.10 | 64.63 |
| IF-B | 92.20 | 84.20 | 66.24 | 88.20 | 66.24 | 77.22 |

The results indicate that the similarity coefficient of IF-B is higher than IF-A, which is consistent with the clinical trial, reflecting the accuracy of evaluation model of this patent to some extent.

EXAMPLE 3

The similarity coefficients of 5 infant formulas fat samples, referred to as IF 1-5, are evaluated with the use of the above evaluation model.

The composition of total fatty acids, *sn-2* fatty acids and triacylglycerols of 5 infant formulas fat samples are given in Table 11~13 below.

Table 11. Fatty acids composition of 5 infant formulas fat samples

| Fatty acids | IF1 | IF2 | IF3 | IF4 | IF5 |
|---|---|---|---|---|---|
| 4:0 | 1.09 | 1.91 | 0 | 2.47 | 0.11 |
| 6:0 | 1.02 | 1.4 | 0.03 | 1.72 | 0.65 |
| 8:0 | 1.04 | 1.29 | 0.84 | 1.06 | 6.41 |
| 10:0 | 2.87 | 2.21 | 0.85 | 2.17 | 4.32 |
| 12:0 | 3.83 | 5.18 | 7.12 | 2.42 | 22.97 |
| 14:0 | 7.91 | 7.59 | 3.3 | 7.98 | 6.48 |
| 14:1(n-5) | 0.33 | 0.74 | 0 | 0.94 | 0.08 |
| 16:0 | 27.94 | 20.33 | 7.56 | 24.86 | 7.01 |
| 16:1(n-7) | 0.55 | 0.76 | 0.15 | 0.9 | 0.15 |
| 18:0 | 8.02 | 6.21 | 3.07 | 8.05 | 2.34 |
| 18:1(n-9) | 24.84 | 27.05 | 50.97 | 23.69 | 30.58 |
| 18:2(n-6) | 14.37 | 18.71 | 19.5 | 17.17 | 15.42 |
| 18:3(n-6) | 0.03 | 0 | 0.04 | 0.04 | 0 |
| 18:3(n-3) | 1.36 | 1.97 | 2.08 | 1.7 | 1.82 |
| 20:0 | 0.21 | 0.13 | 0.36 | 0.21 | 0.12 |
| 20:1(n-9) | 0.06 | 0.1 | 0.44 | 0.13 | 0.12 |
| 20:2(n-6) | 0.02 | 0.05 | 0.03 | 0.06 | 0 |
| 20:3(n-6) | 0.07 | 0 | 0.05 | 0.09 | 0 |
| 20:4(n-6) | 0.23 | 0.08 | 0.53 | 0.36 | 0.05 |
| 22:0 | 0.12 | 0.13 | 0.54 | 0.13 | 0.21 |
| 22:1(n-9) | 0.03 | 0.02 | 0.09 | 0 | 0 |
| 20:5(n-3) | 0.05 | 0.04 | 0.02 | 0.05 | 0 |
| 24:0 | 0.11 | 0.05 | 0.19 | 0.08 | 0.09 |
| 22:5(n-6) | 0.04 | 0 | 0.09 | 0.01 | 0 |
| 24:1(n-9) | 0 | 0.02 | 0.04 | 0 | 0 |
| 22:5(n-3) | 0.05 | 0.06 | 0.02 | 0.04 | 0 |
| 22:6(n-3) | 0.07 | 0.04 | 0.25 | 0.14 | 0.07 |

Table 12. *Sn-2* fatty acids composition of 5 infant formulas fat samples

| Fatty acids | IF1 | IF2 | IF3 | IF4 | IF5 |
|---|---|---|---|---|---|
| 8:0 | 0.5 | 0.41 | 0.16 | 0.62 | 0.31 |
| 10:0 | 2.73 | 1.68 | 0.59 | 2.1 | 1.2 |
| 12:0 | 5.68 | 6.96 | 16.68 | 3.14 | 31.75 |

(continued)

| Fatty acids | IF1 | IF2 | IF3 | IF4 | IF5 |
|---|---|---|---|---|---|
| 14:0 | 12.26 | 11.26 | 2.72 | 13.05 | 4.29 |
| 14:1(n-5) | 0 | 0.8 | 0.02 | 1.14 | 0.06 |
| 16:0 | 25.68 | 20.59 | 3.24 | 24.6 | 1.39 |
| 16:1(n-7) | 1.21 | 1.02 | 0.12 | 1.33 | 0.11 |
| 18:0 | 4.66 | 2.85 | 1.51 | 4.06 | 0.32 |
| 18:1(n-9) | 23.1 | 24.74 | 44.93 | 21.52 | 34.23 |
| 18:2(n-6) | 17.65 | 23.68 | 24.81 | 23.34 | 23.38 |
| 18:3(n-6) | 0 | 0 | 0 | 0.04 | 0 |
| 18:3(n-3) | 1.36 | 2.62 | 3.65 | 1.68 | 2.22 |
| 20:4(n-6) | 0.16 | 0.05 | 0.18 | 0.2 | 0 |
| 22:6(n-3) | 0.08 | 0.05 | 0.13 | 0.09 | 0.09 |

Table 13. Triacylglycerols composition of 5 infant formulas fat samples

| Triacylglycerols | IF1 | IF2 | IF3 | IF4 | IF5 |
|---|---|---|---|---|---|
| BuOM | 1.148 | 1.574 | 0 | 1.269 | 0 |
| CaLaLa | 0.541 | 0 | 1.745 | 0 | 2.79 |
| CoMM+BuMP | 1.281 | 1.192 | 0 | 1.819 | 0 |
| BuOO | 3.104 | 1.145 | 0 | 0.774 | 0 |
| CoOM | 4.482 | 0 | 0 | 0 | 0 |
| BuOP | 0.69 | 2.345 | 0 | 4.128 | 0 |
| LaLaLa | 0 | 0 | 1.837 | 0 | 4.273 |
| CaCaP | 1.148 | 0 | 0 | 0 | 0 |
| CaLaM | 0 | 0.567 | 0 | 1.595 | 0 |
| BuPP | 0.605 | 3.166 | 0 | 5.64 | 0 |
| CoOP | 3.706 | 2.533 | 0 | 2.628 | 0 |
| BuOS | 1.203 | 3.04 | 0 | 8.618 | 0 |
| LaLaM | 0 | 0 | 1.379 | 0 | 2.79 |
| CaLaP | 2.898 | 0 | 0.995 | 0 | 2.478 |
| CoPP | 3.65 | 4.532 | 0 | 8.638 | 0 |
| BuSP | 1.024 | 0 | 0 | 0 | 0 |
| LLLn | 0 | 2.454 | 1.439 | 3.023 | 3.243 |
| CyOO | 0.742 | 0 | 0 | 0 | 0 |
| LaLaO | 3.099 | 0 | 0.078 | 0 | 0.996 |
| CaOM+CyOP | 4.738 | 2.433 | 0 | 5.46 | 0 |
| MMLa | 0 | 0.74 | 0.072 | 2.303 | 0.412 |
| CoPS | 1.124 | 0 | 0 | 0 | 0 |
| LLL | 4.542 | 5.355 | 2.491 | 3.28 | 4.454 |

(continued)

| Triacylglycerols | IF1 | IF2 | IF3 | IF4 | IF5 |
|---|---|---|---|---|---|
| LLnO | 2.365 | 3.821 | 1.677 | 2.936 | 2.212 |
| LLnP | 0 | 0.552 | 0 | 1.25 | 0.183 |
| CaOO | 0.604 | 0 | 0 | 0 | 0 |
| LaOM | 1.277 | 0 | 0 | 0 | 0 |
| CaOP | 1.709 | 0 | 0 | 0 | 0.102 |
| LaMP | 0 | 0 | 0.24 | 0 | 0.478 |
| OLL | 4.862 | 10.573 | 7.721 | 3.054 | 4.896 |
| PLL | 4.542 | 7.136 | 3.604 | 3.963 | 3.54 |
| LaOP | 1.684 | 1.339 | 0.042 | 2.607 | 0.121 |
| MMP+PPLa | 1.533 | 1.172 | 0.068 | 1.745 | 0.21 |
| OOL | 3.171 | 9.501 | 10.043 | 1.946 | 3.783 |
| POL | 0 | 0 | 3.049 | 0 | 2.916 |
| MOO | 6.652 | 6.701 | 0 | 5.199 | 0 |
| PPL | 0 | 0 | 0.239 | 0 | 0.303 |
| MPO | 4.771 | 2.712 | 0 | 4.644 | 0 |
| MPP | 1.129 | 1.011 | 0.033 | 1.682 | 0.04 |
| OOO | 2.309 | 13.001 | 55.698 | 1.206 | 52.495 |
| OOP | 8.154 | 4.096 | 4.367 | 4.509 | 3.798 |
| POP | 9.677 | 2.19 | 0.151 | 5.153 | 0.266 |
| PPP | 0.766 | 0.5 | 0.053 | 1.412 | 0 |
| SOO | 0.538 | 0.634 | 1.648 | 0.509 | 1.372 |
| POS | 1.295 | 0.494 | 0.042 | 1.307 | 0.066 |
| PPS | 0.345 | 0.256 | 0 | 0.554 | 0 |

The similarity coefficients of 5 infant formulas fat samples are evaluated with the use of the above evaluation model. The results are shown in Table 14 below.

Table 14. Similarity coefficients of 5 infant formulas fat samples

| | $G_{III}$ | | | | | | $G_{II}$ | | | $G_I$ |
|---|---|---|---|---|---|---|---|---|---|---|
| | $G_{MAFA}$ | $G_{MIFA}$ | $G_{sn-2MAFA}$ | $G_{sn-2MIFA}$ | $G_{MATAG}$ | $G_{MITAG}$ | $G_{FA}$ | $G_{sn-2FA}$ | $G_{TAG}$ | $G$ |
| IF1 | 88.68 | 22.87 | 71.43 | 47.99 | 64.58 | 95.59 | 55.78 | 59.71 | 80.09 | 65.19 |
| IF2 | 95.96 | -14.82 | 70.09 | 41.87 | 52.16 | 52.44 | 40.57 | 55.98 | 52.30 | 49.62 |
| IF3 | 76.90 | 73.33 | 24.10 | 50.82 | -22.72 | 72.09 | 75.11 | 37.46 | 24.69 | 45.75 |
| IF4 | 96.92 | -18.78 | 70.92 | 37.40 | 69.39 | -18.60 | 39.07 | 54.16 | 25.40 | 39.54 |
| IF5 | 50.55 | -52.24 | 7.28 | 54.04 | -19.04 | 44.27 | -0.84 | 30.66 | 12.61 | 14.14 |

EXAMPLE 4

The structured lipids for human milk fat substrates are produced from extra virgin olive oil, tripalmitin, arachidonic acid and docosahexaenoic acid using Novozym 435 and/or Lipozyme TL IM through one-step or two-steps process. At last four kinds of structured lipids, referred to as SL1-4, are obtained.

The composition of total fatty acids, *sn-2* fatty acids and triacylglycerols of 4 kinds of structured lipids are given in Table 15~17 below.

Table 15. Fatty acids composition of 4 kinds of structured lipids

| Fatty acids | SL1 | SL2 | SL3 | SL4 |
|---|---|---|---|---|
| 8:0 | 0 | 0 | 0.5 | 0.6 |
| 10:0 | 1.04 | 1.02 | 1.11 | 0.7 |
| 12:0 | 0.21 | 0.18 | 0.44 | 0.42 |
| 14:0 | 1.97 | 2.11 | 2.47 | 2.54 |
| 16:0 | 36.69 | 44.23 | 35.23 | 40.07 |
| 16:1(n-7) | 1.03 | 0.87 | 0.85 | 0.84 |
| 18:0 | 2.82 | 2.58 | 3.19 | 3.02 |
| 18:1(n-9) | 43.22 | 38.64 | 38.08 | 37.1 |
| 18:2(n-6) | 6.34 | 5.29 | 5.79 | 4.09 |
| 20:0 | 0.29 | 0.23 | 0.33 | 0.28 |
| 18:3(n-6) | 0.08 | 0.06 | 0.46 | 0.45 |
| 18:3(n-3) | 0.47 | 0.39 | 0.33 | 0.3 |
| 22:0 | 0.16 | 0.12 | 0.28 | 0.27 |
| 20:3(n-3) | 0.16 | 0.11 | 0.55 | 0.54 |
| 20:4(n-6) | 3.67 | 2.97 | 6.23 | 5.95 |
| 22:6(n-3) | 1.53 | 1.39 | 3.71 | 2.6 |

Table 16. *Sn*-2 fatty acids composition of 4 kinds of structured lipids

| Fatty acids | SL1 | SL2 | SL3 | SL4 |
|---|---|---|---|---|
| 14:0 | 0 | 1.12 | 1.44 | 1.24 |
| 16:0 | 52.67 | 56.25 | 50.33 | 55.34 |
| 16:1(n-7) | 0 | 0 | 0.87 | 0.73 |
| 18:0 | 0 | 0 | 2.34 | 1.97 |
| 18:1(n-9) | 39.64 | 34.85 | 34.48 | 33.5 |
| 18:2(n-6) | 6.06 | 6.34 | 4.38 | 3.91 |
| 20:4(n-6) | 2.25 | 1.09 | 4.93 | 4.13 |
| 22:6(n-3) | 0.78 | 0.83 | 2.41 | 2.05 |

Table 17. Triacylglycerols composition of 4 kinds of structured lipids

| Triacylglycerols | SL1 | SL2 | SL3 | SL4 |
|---|---|---|---|---|
| OAO | 0.98 | 0.75 | 1.21 | 0.74 |
| APA | 2.56 | 1.78 | 6.11 | 5.24 |

(continued)

| Triacylglycerols | SL1 | SL2 | SL3 | SL4 |
|---|---|---|---|---|
| OPD | 1.4 | 1.59 | 2.36 | 2.14 |
| ODO | 0.33 | 0.36 | 1.2 | 0.98 |
| LOL | 0 | 0 | 2.07 | 1.44 |
| LPL | 1.46 | 1.14 | 0.66 | 0.87 |
| MPL | 0.84 | 0.72 | 2.35 | 0.88 |
| POLn | 2.13 | 1.5 | 6.03 | 4.56 |
| SMM | 0 | 0 | 1.44 | 2.59 |
| OOL | 3.22 | 1.68 | 2.11 | 2.02 |
| POL | 6.28 | 4.68 | 6.08 | 4.34 |
| PLP | 2.53 | 3.42 | 2.32 | 2.37 |
| PPM | 1.12 | 1.11 | 0.88 | 0.67 |
| OOO | 8.32 | 6.12 | 7.64 | 6.83 |
| OPO | 25.17 | 28.84 | 23 | 25.96 |
| PPO | 31.35 | 33.95 | 24.82 | 28.64 |
| PPP | 4.5 | 10.32 | 4.02 | 6.23 |
| OOS | 1.83 | 0.86 | 1.38 | 1.15 |
| POS | 4.31 | 2.05 | 3.8 | 3.65 |
| PPS | 0.82 | 0.68 | 0.78 | 0.82 |

The similarity coefficients of 4 kinds of structured lipids evaluated with the use of the above evaluation model are shown in Table 18.

Table 18. Similarity coefficients of 4 kinds of structured lipids

| | $G_{III}$ | | | | | | $G_{II}$ | | | $G_I$ |
|---|---|---|---|---|---|---|---|---|---|---|
| | $G_{MAFA}$ | $G_{MIFA}$ | $G_{sn-2MAFA}$ | $G_{sn-2MIFA}$ | $G_{MATAG}$ | $G_{MITAG}$ | $G_{FA}$ | $G_{sn-2FA}$ | $G_{TAG}$ | $G$ |
| SL1 | 68.85 | 55.73 | 24.28 | 48.36 | 39.42 | 96.72 | 62.29 | 36.32 | 68.07 | 55.56 |
| SL2 | 62.84 | 59.31 | 31.84 | 52.38 | 42.67 | 100.00 | 61.08 | 42.11 | 71.34 | 58.17 |
| SL3 | 71.34 | 30.46 | 56.52 | 34.86 | 50.52 | 100.00 | 50.90 | 45.69 | 75.26 | 57.28 |
| SL4 | 66.57 | 36.56 | 55.24 | 39.14 | 47.21 | 100.00 | 51.56 | 47.19 | 73.60 | 57.45 |

The major virtue of this invention is that the evaluation highlights each index without the influence of the content of indexes, and quantifies the differences between dietary fat and human milk fat by the similarity coefficients. Another feature is the ability to grade human milk fat and evaluate the similarity of dietary fat to human milk fat.

It should be stated that the above examples are used only to illustrate the procedures of this invention, but not the limitation to them. Those skilled in this art also will appreciate that while the above examples illustrate the application of this invention, the method is applicable to essentially all substances containing lipids.

**Claims**

1.  **A method for evaluating the similarity between dietary fats and human milk fat,** wherein the composition of major fatty acids, trace fatty acids, major sn-2 fatty acids, trace sn-2 fatty acids, major triacylglycerols and trace triacylglycerols is analysed, and wherein the major fatty acids are the fatty acid that represents more than 1% of the total fatty acids; major sn-2 fatty acids are the fatty acids that represent more than 1% of the total sn-2 fatty acids; major triacylglycerols are the triacylglycerols that represent more than 1% of the total triacylglycerols; trace fatty acids are defined as the fatty acids with less than 1% of the total fatty acids, except for special fatty acids; trace sn-2 fatty acids are defined as the fatty acids with less than 1% of the total sn-2 fatty acids, except for special fatty acids; trace triacylglycerols are defined as the triacylglycerols with less than 1% of the triacylglycerols; special fatty acids are fatty acids that are different from conventional fatty acids due to their special structure of functional group contain odd-chain fatty acids, branched-chain fatty acids, cyclic fatty acids, alkenyl acid, **comprising:**

**(1) calculating the drift rate of each index of dietary fat,** by **comparing the actual values of a** certain index in dietary fat with the range of standard values of the corresponding index in human milk fat by use of the equation:

$$C = \frac{|b - a|}{a}$$

where a represents the standard value of an index, b represents the actual value of the index; and if b is more than $a_{max}$, a is equivalent to $a_{max}$; if b is less than $a_{min}$, a is equivalent to $a_{min}$; C, represents the extent of the determined value of a certain index deviating from the theoretical value range, i.e. shift rate; When b is between $a_{min}$ and $a_{max}$, C is defined as 0, namely, shift rate is equivalent to 0.

(2) establishing a similarity evaluation model, calculated using the equation:

$$G = \sum_{i=1}^{n} \left[ (1 - \frac{|b - a|}{a}) \times 100 \right] \times \frac{d_i}{\sum_{i=1}^{n} d_i}$$

where $d_i$ represents the appointed weight for $i^{th}$ index; $d_i / \sum_{i=1}^{n} d_i$ represents the normalized weight for the $i^{th}$ index after normalization; G represents the similarity coefficient of a certain index of dietary fat; the higher G value, the higher the similarity; and the maximum of G value is 100; conversely, the smaller G value, the lower similarity;

**characterized by** comprising conducting a hierarchical evaluation of multi-grade indexes of dietary fat and calculating the similarity using the equations:

$$G_{III} = \sum_{i=1}^{n} \left[ (1 - \frac{|b - a|}{a}) \times 100 \right] \times \frac{d_i}{\sum_{i=1}^{n} d_i}$$

$$G_{II} = \sum_{i=1}^{n} e_i G_{III}$$

$$G_I = \sum_{i=1}^{n} f_i G_{II}$$

where $G_{III}$, is the similarity coefficient of each grade III index of dietary fat to human milk fat; $G_{II}$ is the similarity coefficient of each grade II index of dietary fat to human milk fat; $e_i$ is the appointed weight for the $i^{th}$ grade III index, $0 \leq e_i \leq 1$; $G_I$, is the similarity coefficient of all grade II index of dietary fat to human milk fat; $f_i$ is the appointed weight for the $i^{th}$ grade II index, $0 < f_i \leq 1$.

2. The method of claim 1 wherein the standard value of a certain index, a, is expressed as mean $\pm$ n times of standard deviation, using the equation $a = \bar{a} \pm n\sigma$, where $\bar{a}$ is the mean, $\sigma$ is the standard deviation.

3. The method of claim 2 wherein the a is the 95% confidence interval and said n=1.96.

4. The method of claim 1 wherein the grade I index is the lipid composition of human milk fat.

5. The method of claim 1 wherein the grade II index contains fatty acids, *sn-2* fatty acids, triacylglycerols and complicated lipids.

6. The method of claim 1, 3, 4 or 5 wherein the fatty acids in grade III index contains major fatty acids, trace fatty acids and special fatty acids, and the *sn*-2 fatty acids in grade III index contain major *sn*-2 fatty acids, trace *sn*-2 fatty acids and special *sn*-2 fatty acids.

7. The method of claim 1, 3, 4 or 5 wherein the triacylglycerols in grade III index comprises major triacylglycerols and trace triacylglycerols.

8. The method of claim 1, 3, 4 or 5 wherein the complicated lipids in grade III index contains phospholipids, sterols, and glycolipids.

**Patentansprüche**

1. Ein Verfahren zur Bewertung der Ähnlichkeit zwischen Nahrungsfetten und menschlichem Milchfett, wobei die Zusammensetzung aus Hauptfettsäuren, Spurenfettsäuren, sn-2-Hauptfettsäuren, sn-2-Spurenfettsäuren, Haupt-Triacylglycerinen und Spuren-Triacylglycerinen analysiert wird, und wobei die Hauptfettsäuren die Fettsäuren sind, die mehr als 1% der gesamten Fettsäuren ausmachen; sn-2-Hauptfettsäuren die Fettsäuren sind, die mehr als 1% der gesamten sn-2-Fettsäuren ausmachen; Haupt-Triacylglycerine die Triacylglycerine sind, die mehr als 1% der gesamten Triacylglycerine ausmachen; Spurenfettsäuren definiert sind als die Fettsäuren, die weniger als 1% der gesamten Fettsäuren ausmachen, ausgenommen spezielle Fettsäuren; sn-2-Spurenfettsäuren definiert sind als die sn-2-Fettsäuren die weniger als 1% der gesamten sn-2-Fettsäuren ausmachen, mit Ausnahme von speziellen Fettsäuren; Spuren-Triacylglycerine definiert sind als die Triacylglycerine die weniger als 1% der Triacylglycerine ausmachen; spezielle Fettsäuren sind Fettsäuren, die sich von herkömmlichen Fettsäuren durch ihre besondere Struktur der funktionellen Gruppe unterscheiden, sie umfassen Fettsäuren mit ungeraden Kettenlängen, verzweigtkettige Fettsäuren, cyclische Fettsäuren, Alkenylsäure, umfassend:

(1) Berechnung der Driftrate jedes Index eines Nahrungsfetts durch Vergleich der tatsächlichen Werte eines bestimmten Index eines Nahrungsfetts mit dem Bereich der Standardwerte des entsprechenden Index im menschlichen Milchfett unter Verwendung der Gleichung:

$$C = \frac{|b - a|}{a}$$

wobei a den Standardwert eines Indexes darstellt, b den tatsächlichen Wert des Indexes; und wenn b größer als $a_{max}$ ist, entspricht a $a_{max}$; wenn b kleiner als $a_{min}$ ist, entspricht a $a_{min}$; C stellt das Ausmaß der Abweichung des ermittelten Wertes eines bestimmten Indexes vom theoretischen Wertebereich dar, d.h. die Verschiebungsrate; wenn b zwischen $a_{min}$ und $a_{max}$ liegt, ist C als 0 definiert, d.h. die Verschiebungsrate ist gleich 0.

(2) Erstellung eines Modells zur Bewertung der Ähnlichkeit, das anhand der folgenden Gleichung berechnet wird:

$$G = \sum_{i=1}^{n}\left[(1 - \frac{|b - a|}{a}) \times 100\right] \times \frac{d_i}{\sum_{i=1}^{n} d_i}$$

wobei $d_i$ das festgesetzte Gewicht für den i-ten Index darstellt; $d_i \Big/ \sum_{i=1}^{n} d_i$ das normalisierte Gewicht für den i-ten Index nach der Normalisierung darstellt; G den Ähnlichkeitskoeffizienten eines bestimmten Index von Nahrungsfett darstellt; je höher der G-Wert, desto höher die Ähnlichkeit; und der maximale G-Wert ist 100; umgekehrt, je kleiner der G-Wert, desto geringer die Ähnlichkeit;

**dadurch gekennzeichnet, dass** die Durchführung einer hierarchischen Bewertung von Indizes mehrerer Grade für Nahrungsfett und die Berechnung der Ähnlichkeit unter Verwendung folgender Gleichungen umfasst ist:

$$G_{III} = \sum_{i=1}^{n} \left[ (1 - \frac{|b - a|}{a}) \times 100 \right] \times \frac{d_i}{\sum_{i=1}^{n} d_i}$$

$$G_{II} = \sum_{i=1}^{n} e_i G_{III}$$

$$G_I = \sum_{i=1}^{n} f_i G_{II}$$

wobei $G_{III}$ der Ähnlichkeitskoeffizient jedes Grad-III-Index des Nahrungsfetts zum menschlichen Milchfett ist; $G_{II}$ der Ähnlichkeitskoeffizient jedes Grad-II-Index des Nahrungsfetts zum menschlichen Milchfett ist; $e_i$ ist das festgesetzte Gewicht für den i-ten Grad-III-Index, $0 \le e_i \le 1$; $G_I$ ist der Ähnlichkeitskoeffizient aller Grad-II-Indexe von Nahrungsfett zu menschlichem Milchfett; $f_i$ ist das festgesetzte Gewicht für den i-ten Grad-II-Index, $0 \le f_i \le 1$.

2. Verfahren nach Anspruch 1, wobei der Standardwert eines bestimmten Index, a, als Mittelwert $\pm$n-facher Standardabweichung unter Verwendung der Gleichung $\alpha = \overline{\alpha} \pm n\sigma$ ausgedrückt wird, wobei $\overline{\alpha}$ der Mittelwert und $\sigma$ die Standardabweichung ist.

3. Verfahren nach Anspruch 2, wobei a das 95%-Konfidenzintervall und genanntes n=1,96 ist.

4. Verfahren nach Anspruch 1, wobei der Grad-I-Index die Lipidzusammensetzung von menschlichem Milchfett ist.

5. Verfahren nach Anspruch 1, wobei der Grad-II-Index Fettsäuren, sn-2-Fettsäuren, Triacylglycerine und komplizierte Lipide enthält.

6. Verfahren nach Anspruch 1, 3, 4 oder 5, wobei die Fettsäuren im Grad-III-Index Hauptfettsäuren, Spurenfettsäuren und spezielle Fettsäuren umfassen, und die sn-2-Fettsäuren im Grad-III-Index sn-2-Hauptfettsäuren, sn-2-Spurenfettsäuren und spezielle sn-2-Fettsäuren umfassen.

7. Verfahren nach Anspruch 1, 3, 4 oder 5, wobei die Triacylglycerine im Grad-III-Index Haupt-Triacylglycerine und Spuren-Triacylglycerine umfassen.

8. Verfahren nach Anspruch 1, 3, 4 oder 5, wobei die komplizierten Lipide im Grad-III-Index Phospholipide, Sterole und Glykolipide umfassen.

**Revendications**

1. Méthode destinée à évaluer la similitude entre les graisses alimentaires et les graisses du lait humain, dans laquelle la composition en acides gras majeurs, en acides gras à l'état de traces, en acides gras *sn*-2 majeurs, en acides gras *sn*-2 à l'état de traces, en triacylglycérols majeurs et en triacylglycérols à l'état de traces est analysée, et où les acides gras majeurs sont les acides gras qui représentent plus de 1% des acides gras totaux ; les acides gras *sn*-2 majeurs sont les acides gras qui représentent plus de 1% des acides gras *sn*-2 totaux ; les triacylglycérols majeurs sont les triacylglycérols qui représentent plus de 1 % des triacylglycérols totaux ; les acides gras à l'état de traces sont définis comme les acides gras représentant moins de 1% des acides gras totaux, à l'exception des acides gras spéciaux ; les acides gras sn-2 à l'état de traces sont définis comme les acides gras représentant moins de 1% des acides gras *sn*-2 totaux, à l'exception des acides gras spéciaux ; les triacylglycérols à l'état de traces sont définis comme les triacylglycérols représentant moins de 1% des triacylglycérols; les acides gras spéciaux sont des acides gras qui sont différents des acides gras conventionnels en raison de la structure particulière de leur groupement fonctionnel, et comprennent les acides gras à chaîne impaire, les acides gras à chaîne ramifiée, les acides gras cycliques, l'acide alcénylique, comprenant :

(1) le calcul du taux de dérive de chaque indice des graisses alimentaires, en comparant les valeurs réelles d'un certain indice dans les graisses alimentaires avec la plage de valeurs standard de l'indice correspondant dans les graisses du lait humain à l'aide de l'équation :

$$C = \frac{|b - a|}{a}$$

dans laquelle

a représente la valeur standard d'un indice, b représente la valeur réelle de l'indice ; et si b est supérieur à $a_{max}$, a est équivalent à $a_{max}$ ; si b est inférieur à $a_{min}$, a est équivalent à $a_{min}$ ; C représente l'ampleur de l'écart de la valeur déterminée d'un certain indice par rapport à la plage de valeurs théoriques, c'est-à-dire le taux de décalage ; lorsque b est compris entre $a_{min}$ et $a_{max}$, C est défini comme étant égal à 0, c'est-à-dire que le taux de décalage est équivalent à 0 ;

(2) l'établissement d'un modèle d'évaluation de la similitude, calculé à l'aide de l'équation :

$$G = \sum_{i=1}^{n} \left[ \left(1 - \frac{|b-a|}{a}\right) \times 100 \right] \times \frac{d_i}{\sum_{i=1}^{n} d_i}$$

dans laquelle

$d_i$ représente le poids désigné pour le $i^{ème}$ indice ; $d_i / \sum_{i=1}^{n} d_i$ représente le poids normalisé pour le $i^{ème}$ indice après normalisation ; G représente le coefficient de similitude d'un certain indice de graisses alimentaires ; plus la valeur de G est élevée, plus la similitude est grande ; et le maximum de la valeur de G est de 100 ; inversement, plus la valeur de G est petite, plus la similitude est faible ;

**caractérisée** comme comprenant la mise en oeuvre d'une évaluation hiérarchique d'indices multigrades de graisses alimentaires et le calcul de la similitude à l'aide des équations :

$$G_{III} = \sum_{i=1}^{n} \left[ \left(1 - \frac{|b-a|}{a}\right) \times 100 \right] \times \frac{d_i}{\sum_{i=1}^{n} d_i}$$

$$G_{II} = \sum_{i=1}^{n} e_i \, G_{III}$$

$$G_{I} = \sum_{i=1}^{n} f_i \, G_{II}$$

où $G_{III}$ est le coefficient de similitude de chaque indice de grade III des graisses alimentaires par rapport aux graisses du lait humain ; $G_{II}$ est le coefficient de similitude de chaque indice de grade II des graisses alimentaires par rapport aux graisses du lait humain ; $e_i$ est le poids désigné pour le $i^{ème}$ indice de grade III, $0 \leq e_i \leq 1$ ; $G_I$ est le coefficient de similitude de tous les indices de grade II des graisses alimentaires par rapport aux graisses du lait humain ; $f_i$ est le poids désigné pour le $i^{ème}$ indice de grade II, $0 \leq f_i < 1$.

2. Méthode dans la revendication 1, dans laquelle la valeur standard d'un certain indice, a, est exprimée sous forme de moyenne ± n fois l'écart type, à l'aide de l'équation $\alpha = \overline{\alpha} \pm n\sigma$, où $\overline{\alpha}$ est la moyenne, $\sigma$ est l'écart type.

3. Méthode selon la revendication 2, dans laquelle le a est l'intervalle de confiance à 95% et ledit *n*=1,96.

4. Méthode selon la revendication 1, dans laquelle l'indice de grade I est la composition lipidique des graisses du lait humain.

5. Méthode selon la revendication 1, dans laquelle l'indice de grade II contient des acides gras, des acides gras *sn*-2, des triacylglycérols et des lipides complexes.

6. Méthode selon la revendication 1, 3, 4 ou 5, dans laquelle les acides gras de l'indice de grade III contiennent des acides gras majeurs, des acides gras à l'état de traces et des acides gras spéciaux, et les acides gras *sn*-2 de l'indice de grade III contiennent des acides gras *sn*-2 majeurs, des acides gras *sn*-2 à l'état de traces et des acides gras *sn*-2 spéciaux.

7. Méthode selon la revendication 1, 3, 4 ou 5, dans laquelle les triacylglycérols de l'indice de grade III comprennent des triacylglycérols majeurs et des triacylglycérols à l'état de traces.

8. Méthode selon la revendication 1, 3, 4 ou 5, dans laquelle les lipides complexes de l'indice de grade III contiennent des phospholipides, des stérols et des glycolipides.

Figure 1

Figure 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Preparation and Characterization of Human Milk Fat Substitutes Based on Triacylglycerol Profiles. **ZOU XIAOQIANG et al.** JOURNAL OF THE AMERICAN OIL CHEMISTS' SOCIETY (JAOCS). SPRINGER, 03 May 2016, vol. 93, 781-792

- **ZOU XIAOOIANG et al.** Model for Human Milk Fat Substitute Evaluation Based on Triacylglycerol Composition Profile. *JOURNAL OF AGRICULTURE AND FOOD CHEMISTRY,* 10 December 2012, vol. 61, 167-175